# EUROPÄISCHE PATENTSCHRIFT

(11) **EP 2 172 442 B1**
(45) Veröffentlichungstag und Bekanntmachung des Hinweises auf die Patenterteilung: **27.07.2016**
(21) Anmeldenummer: 09169430.7
(22) Anmeldetag: 03.09.2009
(51) Int. Cl.: C07C 67/52, C07C 67/54, C07C 69/24, C07C 69/533, C07C 69/58, C07C 69/587, C11C 3/00, C07C 51/43, C07C 51/44, C07C 53/126, C07C 57/03, C07C 57/12, C11C 1/00, C11C 1/10

(54) **Verfahren und Vorrichtung zur Trennung von Carbonsäuren und/oder Carbonsäureestern**
Method and device for separating carboxylic acids and/or carboxylic acid esters
Procédé et dispositif destinés à la séparation d'acides carboniques ou d'ester d'acide carboniques

(30) Priorität: 06.10.2008 EP 08165932
(43) Veröffentlichungstag der Anmeldung: 07.04.2010
(73) Patentinhaber: Sulzer Chemtech AG, 8404 Winterthur (CH)
(72) Erfinder: Fässler, Peter, 8400 Winterthur (CH); Stepanski, Manfred, 9470 Buchs (CH)
(74) Vertreter: Manitz, Finsterwald & Partner GbR

(56) Entgegenhaltungen:
- EP-A- 0 675 100
- EP-A- 0 891 798
- EP-A- 1 394 144
- EP-A- 1 484 309
- WO-A-00/56693
- WO-A-03/038020
- WO-A-03/045891
- US-A- 2 487 000
- US-A- 4 365 079

## Beschreibung

Die Erfindung betrifft eine Vorrichtung zur Trennung von gesättigten und ungesättigten Carbonsäurekomponenten umfassend eine Destillation zur Gewinnung einer Fraktion von ungesättigten Carbonsäurekomponenten.

Das erfindungsgemässe Verfahren kann zur Verarbeitung von Palmöl, Rapsöl, Weizenkeimöl, Sojaöl, Sonnenblumenöl, Erdnussöl, Olivenöl, Kokosbutter, Kokosfett, Maiskeimöl sowie Ölen aus Bohnen, Spargeln, Tomaten, Karotten, Knoblauch, Kohl, Walnüssen, Erdnüssen, Kastanien oder anderen biogenen Ressourcen eingesetzt werden.

Derartige Naturstoffe enthalten ähnlich wie Rohöl eine Vielzahl verschiedener Carbonsäurekomponenten, was nachfolgend schematisch anhand der Zusammensetzung von Palmöl gezeigt werden soll. Unter Carbonsäurekomponenten sollen die Carbonsäuren selbst, beispielsweise eine Fettsäure, sowie die zugehörigen Carbonsäureester verstanden werden, beispielsweise Fettsäuremethylester, Fettsäureethylester oder Isopropylester. Ein derartiges Palmöl besteht im wesentlichen aus Carbonsäurekomponenten, die grösstenteils als Fettsäuren vorliegen, und aus Glyzerin. Die Carbonsäurekomponenten und das Glyzerin können mittels einer Splittervorrichtung bei 280°C und einem Druck von 60 bar voneinander getrennt werden. Die hier weiter interessierenden Fettsäuren, sowie die korrespondierenden Fettsäuremethylester sind typischerweise wie folgt in dem Produktstrom der oben genannten Destillationsvorrichtung enthalten: die leichtsiedenden Komponenten C12 und C<12 mit einem Anteil von 0.2 Gew%, C14 mit einem Anteil von 1-2 Gew%, C16:0 mit einem Anteil von 38-42 Gew%, C16:1 mit einem Anteil von 0.1 Gew% die höhersiedenden Komponenten C18:3 mit einem Anteil von 0.2 bis 0.5 Gew%, C18:2 mit einem Anteil von 7-9 Gew%, C18:1 mit einem Anteil von 37-42 Gew %, C18:0 mit einem Anteil von 4-6 Gew %, die höchstsiedenden Komponenten C20 mit einem Anteil von 0.2 Gew %, sowie C>20 mit einem Anteil von 2-12 Gew %. Als leichtsiedende Komponenten sollen in der Folge Carbonsäurekomponenten mit bis zu 16 Kohlenstoffatomen verstanden werden, das heisst, bevorzugt Carbonsäuren oder Carbonsäureester, insbesondere Fettsäuren mit einem Siedepunkt von bis zu 215°C bei 10 Torr oder entsprechende Fettsäureester. Als höhersiedende Komponenten werden Carbonsäurekomponenten mit 18 Kohlenstoffatomen bezeichnet. Sind die höhersiedenden Komponenten Fettsäuren, fallen darunter gesättigte oder ungesättigte Fettsäuren mit 18 Kohlenstoffatomen. In gleicher Weise kann sich der Begriff höhersiedende Komponenten auf die entsprechenden Fettsäureester beziehen, insbesondere Fettsäuremethylester. Die Siedepunkte dieser Fettsäuren liegen bei 10 Torr zwischen 220 und 230°C. Als höchstsiedende Komponenten werden Carbonsäurekomponenten mit mehr als 18 Kohlenstoffatomen bezeichnet. Handelt es sich bei den höchstsiedenden Carbonsäurekomponenten um gesättigte oder ungesättigte Fettsäuren, fallen unter diesen Begriff gesättigte oder ungesättigte Fettsäuren mit mindestens 20 Kohlenstoffatomen, deren Siedepunkte bei 10 Torr über 230°C liegen. In gleicher Weise kann sich der Begriff höchstsiedende Komponenten auf die entsprechenden Fettsäureester beziehen, insbesondere Fettsäuremethylester.

Es ist bekannt, eine Destillationsvorrichtung zu verwenden, um diese Carbonsäurekomponenten in einer oder mehreren Destillationskolonnen in eine oder mehrere leichtsiedende Komponenten, eine oder mehrere höhersiedende Komponenten sowie eine oder mehrere höchstsiedende Komponenten zu zerlegen. Siehe hierzu auch US 4 365 079, EP 1 394 144, WO 03/038020, WO 00/56693, EP 1 484 309, US 2 847 000, WO 03/045891 und EP 0 675 100. Im vorliegenden Beispiel handelt es sich um die Abtrennung von C12 und Carbonsäurekomponenten mit C<12, das heisst Carbonsäurekomponenten mit weniger als 12 Kohlenstoffatomen in ihrer Summenformel sowie C14, C16:0, C16:1 von den höhersiedenden Komponenten C18:0, C18:1, C18:2 sowie C18:3 und den höchstsiedenden Komponenten umfassend Carbonsäuren mit mehr als 18 Kohlenstoffatomen, also insbesondere C20 und Carbonsäurekomponenten mit mehr als 20

Kohlenstoffatomen in ihrer Summenformel, nachfolgend als C>20 bezeichnet.,. Die in der oben angeführten Kurzformel hinzugefügten Zusätze ":0" oder ":1" oder ":2" stehen für die Anzahl an C-C Doppelbindungen in der Summenformel der entsprechenden Carbonsäurekomponente. ":0" bedeutet somit, dass keine Doppelbindungen in der Summenformel vorhanden sind, das heisst, es handelt sich um eine gesättigte Carbonsäurekomponente. ":1" zeigt an, dass eine Doppelbindung in der Summenformel vorhanden ist, das heisst, es handelt sich um eine einfach ungesättigte Carbonsäurekomponente. Entsprechendes gilt für zwei- und mehrfach ungesättigte Carbonsäurekomponenten.

Eine weitere Trennung der höhersiedenden Komponenten in gesättigte Carbonsäurekomponenten, das heisst insbesondere C18:0 und ungesättigte Carbonsäurekomponenten, das heisst insbesondere C18:1, C18:2 sowie C18:3 voneinander ist auf dem Weg der Destillation praktisch nicht möglich, da die Siedepunkte der vorgenannten Carbonsäurekomponenten einerseits sehr nahe beieinander liegen und andererseits bei zu hohen Sumpftemperaturen eine zumindest teilweise Zersetzung der Carbonsäurekomponenten erfolgen kann. Der Kondensationspunkt von Ölsäure C18:1 mit der Summenformel C₁₇H₃₃COOH, einer einfach ungesättigten, unverzweigten Fettsäure, liegt bei 229°C. Der Kondensationspunkt von Linolsäure C18:2 mit der Summenformel C₁₇H₃₁COOH, einer zweifach ungesättigten, unverzweigten Fettsäure, liegt bei 230°C. Der Kondensationspunkt von Linolensäure C18:3 mit der Summenformel C₁₇H₂₉COOH, einer dreifach ungesättigten, unverzweigten Fettsäure, liegt bei 232°C. Weil insbesondere die ungesättigten Fettsäuren sich thermisch leicht zersetzen, müsste die Destillation mit einer hohen Anzahl Trennstufen im Vakuum durchgeführt werden, was technisch äusserst aufwendig ist. Andere Lösungen, wie beispielsweise Extraktion, haben sich bisher nicht als wirtschaftlich erwiesen.

Ausgehend von diesem Stand der Technik ist es daher eine Aufgabe der Erfindung eine Anlage und ein Verfahren vorzuschlagen, mit welchen Carbonsäurekomponenten, die destillativ aufgrund der vorhergehenden Ausführungen -zumindest in praxistauglicher Weise- nicht mehr trennbar sind, weiter aufzutrennen, sodass auch Carbonsäurekomponenten mit sehr ähnlichen Siedepunkten in einzelne Produktfraktionen getrennt werden können.

Eine weitere Aufgabe der Erfindung besteht darin, die Carbonsäurekomponenten ohne Zusatz von Lösungsmitteln voneinander zu trennen.

Diese Aufgaben werden durch eine Anlage zur Trennung von Carbonsäurekomponenten, umfassend eine Destillationsvorrichtung, in welcher die Carbonsäurekomponenten in eine leichtsiedende Komponente, eine höhersiedende Komponente und eine höchstsiedende Komponente zerlegbar sind, gelöst, wobei die höhersiedende Komponente gesättigte oder ungesättigte Fettsäuren mit 18 Kohlenstoffatomen, deren Siedepunkt bei 10 Torr zwischen 220 und 230°C liegt, und wobei die höchstsiedende Komponente mehr als 18 Kohlenstoffatome aufweist und gesättigte oder ungesättigte Fettsäuren mit mindestens 20 Kohlenstoffatomen umfasst, deren Siedepunkte bei 10 Torr über 230°C liegen, wobei der Destillationsvorrichtung eine Kristallisationsvorrichtung zur Trennung der höhersiedenden Komponente in gesättigte und ungesättigte Carbonsäurekomponenten nachgeschaltet ist, wobei die Kristallisationsvorrichtung als eine Schichtkristallisationsvorrichtung ausgebildet ist, wobei die Schichtkristallisationsvorrichtung senkrechte oder geneigte Kristallisationsflächen zur Bildung eines Kristallisats an hochschmelzenden gesättigten Carbonsäurekomponenten enthält, und wobei die senkrechten oder geneigten Kristallisationsflächen zueinander in einem Abstand angeordnet sind, sodass eine Mutterlauge an niedrig schmelzenden ungesättigten Carbonsäurekomponenten abführbar ist, wobei eine für die flüssige Mutterlauge durchlässige siebartige Stützstruktur zwischen benachbarten Kristallisationsflächen vorgesehen ist, wobei stromaufwärts der Destillationsvorrichtung und der Kristallisationsvorrichtung eine Veresterungsvorrichtung angeordnet ist, und wobei die Veresterungsvorrichtung einen Reaktor umfasst, der einen heterogenen Katalysator enthält.

Ein weiterer Gegenstand der vorliegenden Erfindung ist ein Verfahren zur Trennung von Carbonsäurekomponenten, umfassend eine Destillationsvorrichtung, in welcher in einem ersten Schritt die Carbonsäurekomponenten in eine leichtsiedende Komponente, eine höhersiedende Komponente und eine höchstsiedende Komponente, wobei die höhersiedende Komponente gesättigte oder ungesättigte Fettsäuren mit 18 Kohlenstoffatomen deren Siedepunkt bei 10 Torr zwischen 220 und 230°C liegt, und wobei die höchstsiedende Komponente mehr als 18 Kohlenstoffatome aufweist und gesättigte oder ungesättigte Fettsäuren mit mindestens 20 Kohlenstoffatomen umfasst, deren Siedepunkte bei 10 Torr über 230°C liegen, in einer zuvor beschriebenen Anlage zerlegt werden, in einem zweiten Schritt die höhersiedende Komponente einer Schichtkristallisationsvorrichtung zugeführt wird und in einem dritten Schritt die höhersiedende Komponente in der Schichtkristallisationsvorrichtung in ein Kristallisat, welches gesättigte Carbonsäurekomponenten enthält, und in eine Mutterlauge, welche im wesentlichen ungesättigte Carbonsäurekomponenten enthält, getrennt wird.

Die Kristallisationsvorrichtung ist als eine Schichtkristallisationsvorrichtung ausgebildet, was den besonderen Vorteil hat, dass die Kristallisation ohne Zusatz von Lösungsmitteln erfolgen kann. Eine derartige Kristallisationsvorrichtung für die Reinigung von Paraffinen, Ölen, Fetten und Wachsen ist beispielsweise in der EP 0 891 798 offenbart. Erfindungsgemäß enthält die Schichtkristallisationsvorrichtung senkrechte oder geneigte Kristallisationsflächen zur Bildung eines Kristallisats an niedrigschmelzenden gesättigten Carbonsäurekomponenten. Diese senkrechten oder geneigten Kristallisationsflächen sind zueinander in einem Abstand angeordnet, sodass eine Mutterlauge an höherschmelzenden ungesättigten Carbonsäurekomponenten abführbar ist, wobei eine für die flüssige Mutterlauge durchlässige siebartige Stützstruktur zwischen benachbarten Kristallisationsflächen vorgesehen ist.

Vor der Destillationsvorrichtung und der Kristallisationsvorrichtung ist eine Veresterungsvorrichtung angeordnet. Die Durchführung einer Veresterung in dieser Veresterungsvorrichtung hat den besonderen Vorteil, dass die maximale Betriebstemperatur in der Destillationskolonne erniedrigt werden kann. Eine Reduktion der Betriebstemperatur hat den Vorteil, das thermisch nur begrenzt stabile Carbonsäurekomponenten ohne die Gefahr einer Degradation in der Destillationsvorrichtung getrennt werden können.

Die Veresterungsvorrichtung umfasst bevorzugt einen Reaktor, der einen heterogenen Katalysator enthält. Die Verwendung eines heterogenen Katalysators, der in fester Phase vorliegt hat den Vorteil, dass der Katalysator einfach ausgetauscht werden kann und eine Trennung von der flüssigen Phase problemlos erfolgen werden kann.

Das Verfahren zur Trennung von Carbonsäurekomponenten umfasst eine Destillationsvorrichtung in welcher in einem ersten Schritt die Carbonsäurekomponenten in eine leichtsiedende Komponente, eine höhersiedende Komponente und eine höchstsiedende Komponente zerlegt werden, in einem zweiten Schritt die höhersiedende Komponente einer Kristallisationsvorrichtung zugeführt wird und in einem dritten Schritt die höhersiedende Komponente in der Kristallisationsvorrichtung in ein Kristallisat, welches im wesentlichen gesättigte Carbonsäurekomponenten enthält, und in eine Mutterlauge welche im wesentlichen ungesättigte Carbonsäurekomponenten enthält, getrennt wird.

Die Carbonsäurekomponenten werden in einer Veresterungsvorrichtung zumindest teilweise in Carbonsäureester umgewandelt, wobei die Veresterungsvorrichtung insbesondere mittels heterogener Katalyse betrieben wird. Die leichtsiedende Komponente kann ein Gemisch von mehreren Carbonsäurekomponenten mit unterschiedlicher Anzahl an Kohlenstoffatomen umfassen. Ein erster Teil des Gemischs der leichtsiedenden Komponenten wird in einer ersten Destillationskolonne als Kopfprodukt abgezogen, ein zweiter Teil des Gemisches der leichtsiedenden Komponenten sowie die höhersiedende Komponente und die höchstsiedende Komponente fällt als Sumpfprodukt der ersten Destillationskolonne an, wobei das Sumpfprodukt der ersten Destillationskolonne einer zweiten Destillationskolonne zugeführt wird. In der zweiten Destillationskolonne wird der zweite Teil des Gemisches der leichtsiedenden Komponenten als Kopfprodukt abgezogen, wobei die höhersiedende Komponente und die höchstsiedende Komponente als Sumpfprodukt der zweiten Destillationskolonne anfällt. Das Sumpfprodukt der zweiten Destillationskolonne wird einer dritten Destillationskolonne zugeführt, wobei die höhersiedende Komponente als Kopfprodukt der dritten Destillationskolonne der Kristallisationsvorrichtung zugeführt wird, sowie als Sumpfprodukt der dritten Destillationskolonne die höchstsiedende Komponente abgezogen wird.

Im Folgenden wird die Erfindung sowohl in apparativer als auch in verfahrenstechnischer Hinsicht anhand besonders bevorzugter Ausführungsbeispiele und anhand der nachfolgenden Figuren näher erläutert. Es zeigen teilweise in schematischer Darstellung
- Fig. 1:: ein erstes Ausführungsbeispiel einer Anlage zur Durchführung des erfindungsgemässen Verfahrens
- Fig. 2:: ein zweites Ausführungsbeispiel einer Anlage zur Durchführung des erfindungsgemässen Verfahrens
- Fig. 3:: ein Detail betreffend die Veresterung gemäss Fig. 2
- Fig. 4:: ein drittes Ausführungsbeispiel einer Anlage zur Durchführung des erfindungsgemässen Verfahrens
- Fig. 5:: eine Ansicht einer Kristallisationsvorrichtung
- Fig. 6:: einen Schnitt durch die Kristallisationsvorrichtung gemäss Fig. 5
- Fig. 7:: ein Detail der Kristallisationsvorrichtung gemäss Fig. 5 oder Fig. 6
- Fig. 8:: ein Detail der Kristallisationsvorrichtung gemäss Fig. 5 oder Fig. 6
- Fig. 9-12:: eine Darstellung verschiedener Stadien der Kristallisation, und
- Fig. 13:: eine Modifikation der Kristallisationsvorrichtung.

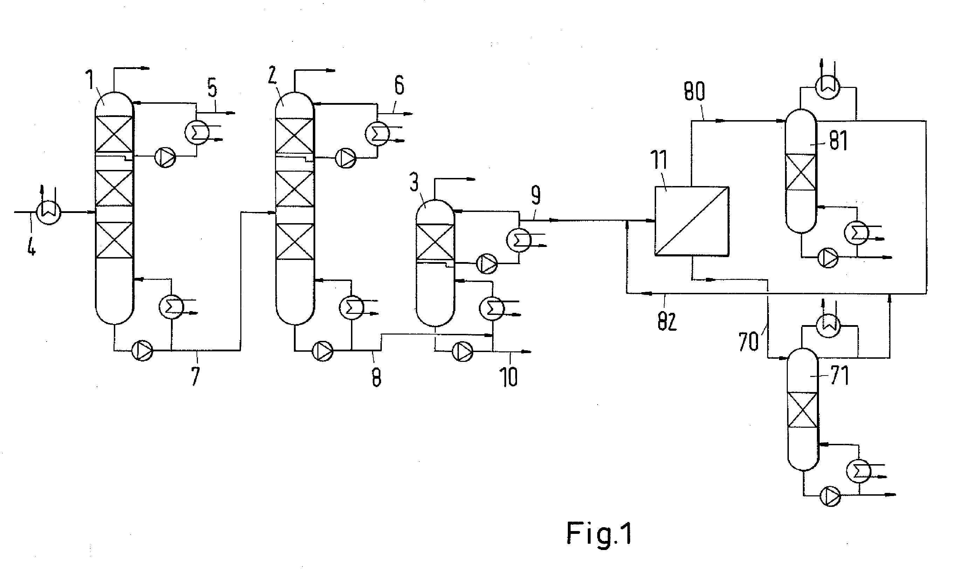

Die in Fig. 1 dargestellte Anlage zur Trennung von Carbonsäurekomponenten geht von einem Einsatzgemisch 4 aus, welches die Carbonsäurekomponenten enthält. Die Carbonsäurekomponenten sind erhältlich aus Carbonsäuren pflanzlicher oder tierischer Herkunft. Insbesondere sind die Carbonsäurekomponenten als Destillat einer Trennung eines Öls, beispielsweise eines Palmöls, erhältlich und werden mittels einer Destillationsvorrichtung, umfassend eine oder mehrere Destillationskolonnen (1,2,3) weiter verarbeitet. In der vorgenannten Destillationsvorrichtung werden die leichtsiedenden Komponenten von den höhersiedenden Komponenten und den höchstsiedenden Komponenten getrennt. Die Trennung kann in grösseren Anlagen in mehreren Destillationskolonnen ablaufen, die Trennaufgabe somit in mehrere Teilschritte zerlegt werden.

In Fig. 1 werden in einer ersten Destillationskolonne 1 ein erster Teil der leichtsiedenden Komponenten, nämlich insbesondere C12 , C14 und allfällige Anteile von C<12 als Kopfprodukt 5 abgetrennt. In einer zweiten Destillationskolonne 2 wird ein zweiter Teil der leichtsiedenden Komponenten, C16, als Kopfprodukt 6 abgetrennt. Das Sumpfprodukt 7 der Destillationskolonne 1 enthält den zweiten Teil der leichtsiedenden Komponenten, sowie die höhersiedenden Komponenten und höchstsiedenden Komponenten, insbesondere Carbonsäurekomponenten mit einer Anzahl Kohlenstoffatome ab C16.

Üblicherweise herrscht im Sumpf der ersten Destillationskolonne 1 eine Temperatur von maximal 195°C und ein Druck von maximal 2 bar. Die erste Destillationskolonne 1, ist vorteilhafterweise als gepackte Kolonne ausgeführt. In einer besonders bevorzugten Ausführungsform enthält die erste Destillationskolonne 1 strukturierte Packungen mit Kreuzkanalstruktur, wie sie beispielsweise in der US4455339 oder der US3785620 beschrieben sind.

Gemäss Fig. 1 wird der zweite Teil der leichtsiedenden Komponenten als Kopfprodukt 6 der zweiten Destillationskolonne 2 erhalten. Das Sumpfprodukt 8 der zweiten Destillationskolonne 2 enthält alle höhersiedenden Komponenten, insbesondere Carbonsäurekomponenten mit 18 Kohlenstoffatomen, also C18:0, C18:1, C18:2, C18:3 sowie die höchstsiedenden Komponenten ab C20. Üblicherweise liegt im Sumpf der zweiten Destillationskolonne 2 im Betrieb eine Temperatur von maximal 220°C und ein Druck von maximal 1,5 bar an. Die zweite Destillationskolonne 2 ist vorteilhafterweise als gepackte Kolonne ausgeführt. In einer besonders bevorzugten Ausführungsform enthält die zweite Destillationskolonne 2 strukturierte Packungen mit Kreuzkanalstruktur wie oben in Zusammenhang mit der ersten Destillationskolonne 1 beschrieben worden ist.

Selbstverständlich können der erste und der zweite Teil der leichtsiedenden Komponenten auch als Teilströme an unterschiedlichen Orten derselben Destillationskolonne abgezogen werden und die höhersiedenden Komponenten als ein weiterer Teilstrom abgezogen werden, sowie die höchstsiedenden Komponenten als Sumpfprodukt dieser Destillationskolonne abgezogen werden, was in den Figuren nicht dargestellt ist. Es ist auch möglich, die höhersiedenden und höchstsiedenden Komponenten als Sumpfprodukt dieser Destillationskolonne einer weiteren Destillationskolonne zuzuführen, welche der nachfolgend beschriebenen dritten Destillationskolonne 3 entspricht.

In der dritten Destillationskolonne 3 werden die höhersiedenden Komponenten von den höchstsiedenden Komponenten, also insbesondere Carbonsäurekomponenten mit mehr als 20 Kohlenstoffatomen, abgetrennt. Die höchstsiedenden Komponenten werden als Sumpfprodukt 10 der dritten Destillationskolonne 3 abgezogen.

In Abhängigkeit von der Herkunft des Einsatzstroms können diese höchstsiedenden Komponenten noch Wertstoffe enthalten. Diese Wertstoffe können anschliessend in nachfolgenden Prozessschritten isoliert werden, beispielsweise zur Gewinnung von Vitamin E.

Das Kopfprodukt 9 der dritten Destillationskolonne 3 wird in die Kristallisationsvorrichtung 11 eingespeist. Dieses Kopfprodukt enthält die höhersiedenden Komponenten, also insbesondere Carbonsäurekomponenten mit 18 Kohlenstoffatomen, welche bevorzugt eine gesättigte Carbonsäurekomponente, das heisst insbesondere C18:0, sowie zumindest eine der ungesättigten Carbonsäurekomponenten, das heisst insbesondere C18:1, C18:2 sowie C18:3, enthält, welche mittels der Kristallisationsvorrichtung in die gesättigte Carbonsäurekomponente und die ungesättigten Carbonsäurekomponenten aufgetrennt werden. Optional können noch Reinigungsschritte für die Mutterlauge, also die ungesättigten Carbonsäurekomponenten sowie für das Kristallisat, das hauptsächlich die gesättigte Carbonsäurekomponente enthält, vorgesehen werden. Hier erfolgt eine Reinigung mittels Strippen aus der Flüssigphase.

In Fig. 1 ist weiters ein Stripper 71 für die Mutterlauge 70 gezeigt, sowie ein Stripper 81 für das Kristallisat 80. Das Kristallisat 80 kann noch Spuren der ungesättigten Carbonsäurekomponenten enthalten. Um diese Spuren der ungesättigten Carbonsäurekomponenten abzutrennen und die gesättigte Carbonsäurekomponente zu isolieren, wird das Kristallisat 80 in den Kopfbereich des Strippers 81 eingebracht. Der Stripper 81 wird mit einem Strippmittel, insbesondere mit Aceton, betrieben, in welchem sich die ungesättigten Carbonsäurekomponenten, welche üblicherweise leichtsiedend sind, anreichern. Ein Strom 82, der das Strippmittel, sowie die ungesättigten Carbonsäurekomponenten enthält, wird vom Kopf des Strippers 81 zur Kristallisationsvorrichtung 11 rückgeführt. Die Mutterlauge 70 kann im Stripper 71 von dem leichtersiedenden Strippmittel beispielsweise durch Destillation getrennt werden. Das Strippmittel wird als Kopfprodukt dieser Kolonne erhalten und ebenfalls in die Kristallisationsvorrichtung 11 rückgeführt. In der Darstellung gemäss Fig. 1 wird das Kopfprodukt des Strippers 71 dem Strom 82, der das Strippmittel enthält, zugeführt.

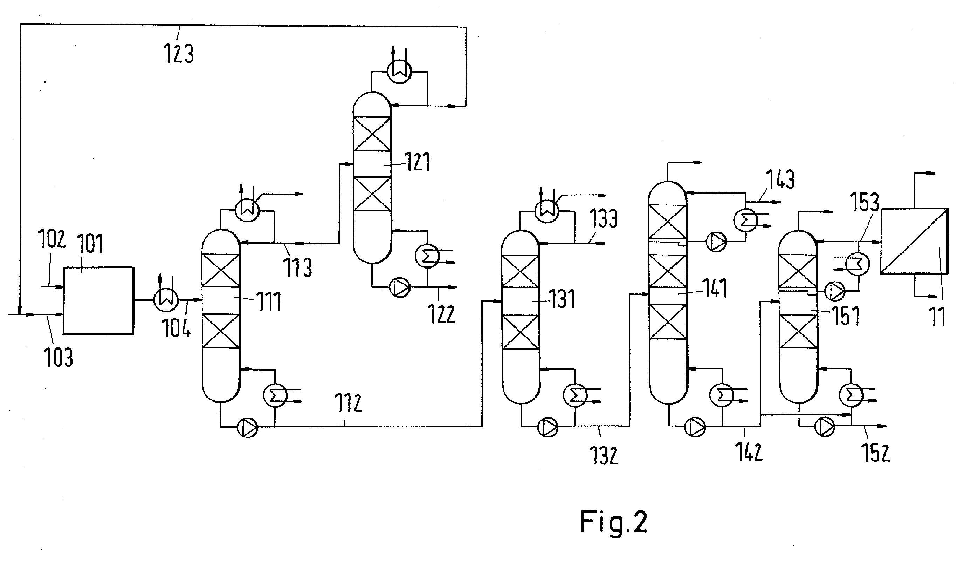

Fig. 2 zeigt eine zweite Verfahrensvariante, gemäss welcher die Carbonsäurekomponenten vor der destillativen Trennung zur Durchführung einer Veresterung einer Veresterungsvorrichtung 101 zugeführt werden. Die Veresterung erfordert die Zugabe von einem Alkohol, beispielsweise Methanol, um die entsprechende Carbonsäurekomponente in den oder die zugehörigen Carbonsäureester umzuwandeln. Die Veresterung kann beispielsweise mittels eines Verfahrens gemäss US 7 091 367 B2 durchgeführt werden. Das Verfahren wird gemäss Fig. 2 in einer Destillationskolonne durchgeführt, welche eine Packung enthält, die zur Durchführung einer heterogenen Katalyse geeignet ist. Als Reaktionsprodukte der heterogenen Katalyse werden Carbonsäureester, insbesondere Carbonsäuremethylester, erhalten. Der Vorteil der Umwandlung der Carbonsäurekomponenten in Carbonsäureester liegt in dem um durchschnittlich 20°C niedrigeren Siedepunkt von Carbonsäureestern, verglichen mit dem Siedepunkt der zugehörigen Carbonsäurekomponenten des Einsatzgemisches.

In der Darstellung gemäss Fig. 2 handelt es sich bei der Veresterungsvorrichtung 101 um einen Kontaktapparat, in welchem die Carbonsäurekomponenten 102, also insbesondere Fettsäuren, mit einem Alkohol 103 in Kontakt gebracht werden. In dem Kontaktapparat befinden sich Einbauten, an deren Oberfläche die Carbonsäurekomponenten und der Alkohol miteinander reagieren. Der Katalysator ist insbesondere in einem Festbettreaktor angeordnet, beispielsweise in einem Rohr, welches mit dem Katalysator befüllt ist. Nach einer Variante kann auch eine homogene Katalyse zum Einsatz kommen, das heisst, in diesem Fall liegt der Katalysator in flüssiger Phase vor. Eine gute Durchmischung wird in diesem Fall beispielsweise in einem Rührkessel erzielt. Die Reaktionsprodukte 104 der Veresterung werden zur Abtrennung von überschüssigem Alkohol einer Destillationskolonne 111 zugeführt. Bei den Reaktionsprodukten handelt es sich bei Verwendung von Methanol als Beispiel für einen Alkohol 103 im wesentlichen um Carbonsäureester, insbesondere Carbonsäuremethylester, im Fall der Verwendung eines Einsatzgemisches aus Fettsäuren um die entsprechenden Fettsäuremethylester. Die Destillationskolonne 111 ist vorteilhafterweise als eine Packungskolonne ausgebildet. Die höhersiedenden Carbonsäureester werden als Sumpfprodukt 112 abgezogen, der Alkohol, hier Methanol 113, wird als Kopfprodukt abgezogen. Für das Methanol kann eine weitere Reinigung erforderlich sein, wenn ein zu hoher Anteil an wässriger Phase vorliegt und das Methanol wieder der Veresterungsvorrichtung zugeführt wird.

In Fig. 2 ist dieser Reinigungsschritt dargestellt, der in einer Destillationskolonne 121 durchgeführt wird. Die Destillationskolonne zur Abtrennung der wässrigen Phase 122 von dem als Kopfprodukt 123 abziehbaren Methanol ist ebenfalls vorteilhafterweise als Packungskolonne ausgebildet. Das Gemisch an Carbonsäureestern, wird dann ähnlich weiterverarbeitetet, wie es in Zusammenhang mit Fig. 1 für die Carbonsäurekomponenten beschrieben worden ist.

Bei der Trennung von Carbonsäureestern, das heisst insbesondere Fettsäuremethylestern (FAME) geht man von einem Einsatzgemisch aus, welches Carbonsäureester enthält, insbesondere die Methylester der Fettsäuren C12, C14, C16, C18:0, C18:1, C18:2 sowie C18:3. Zur Vereinfachung soll nachfolgend für die Fettsäuremethylester die Kurzschreibweise FAME (für fatty acid methyl ester) verwendet werden.

In einer Destillationsvorrichtung, welche gemäss Fig. 2 die Destillationskolonnen 131, 141, 151 umfasst, werden die leichtsiedenden Komponenten, das heisst insbesondere die FAME der Fettsäuren C12, C14, C16 von der höhersiedenden Komponenten, das heisst insbesondere der FAME C18, das heisst C18:0, C18:1, C18:2 sowie C18:3 und den höchstsiedenden Komponenten, das heisst insbesondere der FAME mit mehr als 18 Kohlenstoffatomen getrennt. Selbstverständlich ist die Destillationsvorrichtung auch für andere Carbonsäureester geeignet, in der Folge soll jedoch stellvertretend für die Klasse der Carbonsäureester die Anwendung auf die besonders bevorzugten Fettsäuremethylester (fatty acid methyl esters oder kurz FAME) im Detail beschrieben werden. Die Destillationsvorrichtung umfasst eine erste Destillationskolonne 131, um einen ersten Teil der leichtsiedenden Komponenten, nämlich die C12 sowie C14 und allfällige FAME mit C>12 als Kopfprodukt 133 abzutrennen. Das Sumpfprodukt 132 der ersten Destillationskolonne 131 enthält den zweiten Teil der leichtsiedenden Komponenten, nämlich die FAME C16, sowie die höhersiedende Komponente der FAME und die höchstsiedende Komponente der FAME. In einer zweiten Destillationskolonne 141 wird der zweite Teil der leichtsiedenden Komponenten, insbesondere der C16 FAME als Kopfprodukt 143 vom Sumpfprodukt 132, welches die höhersiedenden und höchstsiedenden Komponenten enthält, abgetrennt. Üblicherweise liegt im Sumpf der ersten Destillationskolonne 131 eine Temperatur von maximal 175°C und ein Druck von maximal 2 bar vor. Die erste Destillationskolonne 131 ist vorteilhafterweise als gepackte Kolonne ausgeführt. In einer besonders bevorzugten Ausführungsform enthält die erste Destillationskolonne eine strukturierte Packung mit Kreuzkanalstruktur, wie sie beispielsweise in der US4455339 oder der US3785620 beschrieben sind.

Gemäss Fig. 2 wird der zweite Teil der leichtsiedenden Komponenten als Kopfprodukt 143 einer zweiten Destillationskolonne 141 erhalten. Das Sumpfprodukt 142 enthält die höhersiedende Komponente sowie die höchstsiedende Komponente. Im Sumpf der zweiten Destillationskolonne 141 liegt eine Temperatur von maximal 200°C und ein Druck von maximal 1,25 bar vor. Die zweite Destillationskolonne 141, ist vorteilhafterweise als gepackte Kolonne ausgeführt. In einer besonders bevorzugten Ausführungsform enthält die zweite Destillationskolonne 141 strukturierte Packungen mit Kreuzkanalstruktur, wie oben in Zusammenhang mit der ersten Destillationskolonne 131 beschrieben worden ist.

Selbstverständlich können die Kopfprodukte 133 und 143 auch als Teilströme an unterschiedlichen Orten derselben Destillationskolonne abgezogen werden, was zeichnerisch nicht dargestellt ist.

In einer dritten Destillationskolonne 151 werden die höhersiedenden Komponenten von den höchstsiedenden Komponenten getrennt. Die höchstsiedenden Komponenten werden als Sumpfprodukt 152 der dritten Destillationskolonne abgezogen. In Abhängigkeit von der Herkunft des Einsatzstroms kann die höchstsiedende Komponente in weiteren Prozessschritten zu Wertstoffen isoliert werden, beispielsweise zur Gewinnung von Vitamin E. Das Kopfprodukt 153 der dritten Destillationskolonne 151 wird in die Kristallisationsvorrichtung 11 eingespeist. Dieses Kopfprodukt 153 enthält die höhersiedende Komponente enthaltend insbesondere die FAME der Carbonsäurekomponenten C18:0, C18:1, C18:2 sowie C18:3, welche durch Kristallisation in die gesättigte Carbonsäurekomponente, insbesondere die FAME der Carbonsäurekomponente C18:0 und die ungesättigten Carbonsäurekomponenten, insbesondere die FAME der C18:1, C18:2 sowie C18:3, aufgetrennt werden. Optional können noch Reinigungsschritte für die Mutterlauge, also der ungesättigten Carbonsäurekomponenten sowie für das Kristallisat, das hauptsächlich die gesättigte Carbonsäurekomponente enthält, vorgesehen werden, wie schon in Fig. 1 beschrieben worden ist, hier aber nicht näher dargestellt ist.

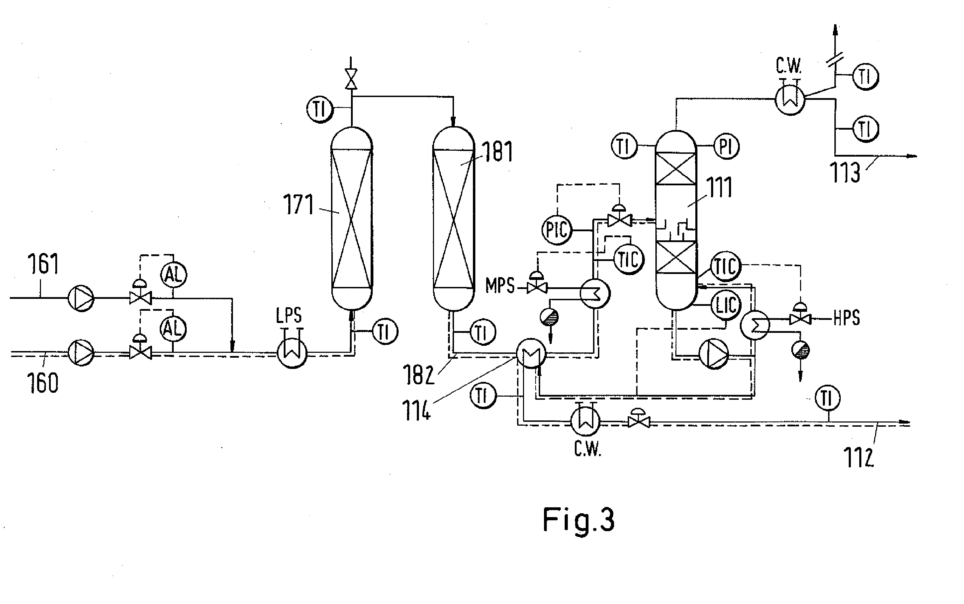

Fig. 3 zeigt eine Anlage zur Herstellung von FAME durch Veresterung eines Fettsäuren enthaltenden Einsatzstroms 160. Dieser Einsatzstrom 160 wird gemeinsam mit einem einen Alkohol enthaltenden Strom 161 einem Reaktor zugeführt. Der Alkohol des Stroms 161 kann insbesondere einen linearen oder verzweigten C1-C10-Monoalkanol wie beispielsweise Methanol, Ethanol, Propanol, Butanol, Pentanol, Hexanol sowie deren Isomere umfassen, auch iso-Propanol oder 2-Ethylhexanol, oder C2-C5 - Di- oder Trialkanole, wie Glycerin, Ethandiol, 1,2 Propandiol, 1,3-Propandiol, Butandiol, Pentandiol, deren Isomere sowie deren Halbester. Der Einsatzstrom 160, sowie der den Alkohol enthaltende Strom 161 werden getrennt oder gemeinsam einem Reaktor zugeführt, der in Fig. 3 aus zwei hintereinander geschalteten Packungskolonnen 171, 181 besteht. Die Packungen enthalten einen Katalysator, der entweder als homogener Katalysator in Lösung vorliegt oder als heterogener Katalysator als Feststoff auf der Packung oder in einem Rohr angebracht ist. Bei der heterogenen Katalyse kommen besonders bevorzugt Festbettreaktoren zum Einsatz. Als Katalysatoren können basische oder saure Anionen- oder Kationentauscher auf organischer oder anorganischer Basis verwendet werden. Alternativ können saure Tonerden, Zeolithe, Bleicherden, Katalysatoren auf der Basis von Übergangsmetalloxiden, wie TiO₂ oder ZrO₂ oder organofunktionellen Polysiloxanen zum Einsatz kommen. Der Produktstrom 182 umfasst somit den überschüssigen Alkohol, Wasser, die FAME sowie Tocopherole und Glyceride. Der Produktstrom 182 wird in die Destillationskolonne 111 eingebracht, in welcher die leichtersiedenden FAME, Alkohole und Wasser von den schwerersiedenden FAME, Tocopherolen und Glyceriden getrennt werden. Die Destillationskolonne 111 ist gleich oder entsprechend der in Fig. 2 dargestellten Destillationskolonne ausgebildet, daher auch mit demselben Bezugszeichen versehen. Zusätzlich ist in Fig. 3 noch gezeigt, dass das Sumpfprodukt 112 durch einen Wärmetauscher 114 geführt wird, um den FAME Produktstrom 182 vorzuheizen, wodurch eine energiesparende Prozessführung ermöglicht ist.

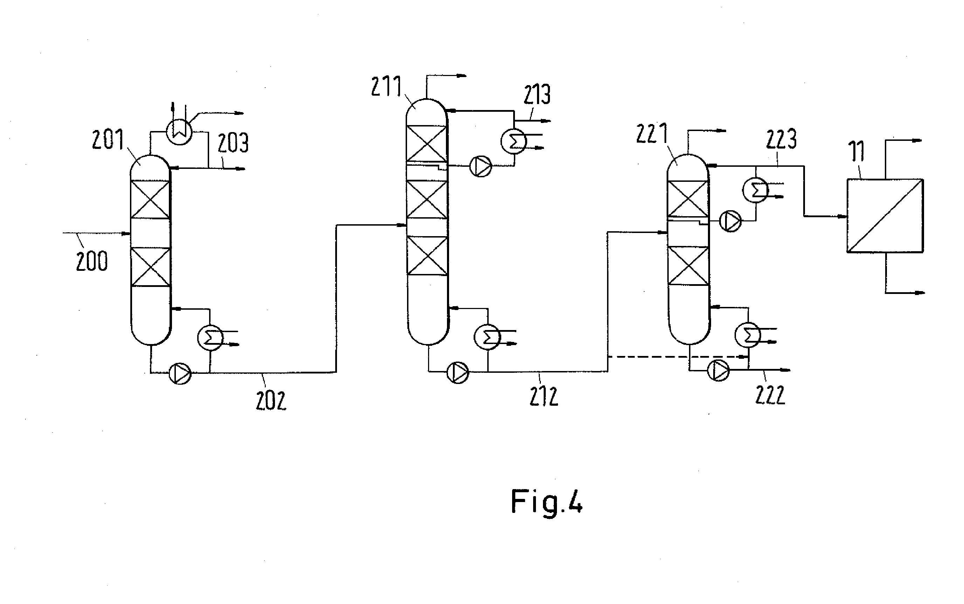

Fig. 4 zeigt ein Verfahren, bei welchem als Einsatzgemisch 200 ein Biodiesel verwendet wird. Dieses Einsatzgemisch 200 enthält Carbonsäurekomponenten wie das Einsatzgemisch 1 gemäss Fig. 1, also Carbonsäuren sowie auch Carbonsäureester wie das Einsatzgemisch 112 der Fig. 2. Bei den Carbonsäuren handelt es sich insbesondere um Fettsäuren und den Carbonsäureestern insbesondere um Fettsäuremethylester. Die Destillationsschritte entsprechen daher den in Fig. 1 sowie Fig. 2 gezeigten Destillationsschritten. Eine erste Destillationskolonne 201 entspricht der ersten Destillationskolonne 1 oder 131 (siehe Fig. 1 und Fig. 2), um einen ersten Teil des Gemisches an leichtsiedenden Komponenten, die insbesondere C12 sowie C14 und allfällige leichter siedende Fettsäuren und/oder FAME enthalten, als Kopfprodukt 203 abzutrennen (entspricht Strom 5,133). Das Sumpfprodukt 202 (entspricht Strom 7 bzw. 132) der Destillationskolonne 201 enthält den zweiten Teil des Gemisches an leichtsiedenden Komponenten, die Fettsäuren und/oder FAME C16, sowie die höhersiedenden Komponenten, insbesondere Fettsäuren oder/und FAME mit 18 Kohlenstoffatomen, also die gesättigten und ungesättigten Fettsäuren C18:0, C18:1, C18:2, C18:3, sowie die höchstsiedenden Komponenten, insbesondere die Fettsäuren und/oder FAME mit mehr als 18 Kohlenstoffatomen, das heisst C>18. In einer zweiten Destillationskolonne 211 (entspricht Destillationskolonne 2 beziehungsweise 141) wird der zweite Teil des Gemisches an leichtsiedenden Komponenten als Kopfprodukt 213 (entspricht Strom 6,143). von den restlichen Komponenten des Sumpfprodukts 212 (entspricht Strom 8,142) abgetrennt. Üblicherweise liegt die Temperatur im Sumpf der ersten Destillationskolonne bei maximal 175°C und der Druck bei maximal 2 bar. Die erste Destillationskolonne 201 ist vorteilhafterweise als gepackte Kolonne ausgeführt, wie vorher beschrieben worden ist.

Gemäss Fig. 4 wird der zweite Teil des Gemisches an leichtsiedenden Komponenten als Kopfprodukt 213 der Destillationskolonne 211 erhalten. Das Sumpfprodukt 212 enthält die höhersiedenden und höchstsiedenden Komponenten. Üblicherweise liegt die Temperatur im Sumpf der zweiten Destillationskolonne 211 bei maximal 200°C und der Druck bei maximal 1,25 bar. Die zweite Destillationskolonne 211 ist vorteilhafterweise als gepackte Kolonne ausgeführt. In einer besonders bevorzugten Ausführungsform enthält die zweite Destillationskolonne strukturierte Packungen mit Kreuzkanalstruktur, wie oben in Zusammenhang mit der ersten Destillationskolonne beschrieben. Selbstverständlich können die beiden Kopfprodukte 203 und 213 auch als Teilströme an unterschiedlichen Orten derselben Destillationskolonne abgezogen werden, was zeichnerisch nicht dargestellt ist.

In der dritten Destillationskolonne 221 (entspricht Destillationskolonne 3, 151) wird höhersiedende Komponente von der höchstsiedenden Komponente getrennt. Die höchstsiedende Komponente wird als Sumpfprodukt 222 (entspricht Strom 10,152) der dritten Destillationskolonne abgezogen. In Abhängigkeit von der Herkunft des Einsatzstroms kann die höchstsiedende Komponente in weiteren Prozessschritten zu Wertstoffen isoliert werden, beispielsweise zur Gewinnung von Vitamin E. Das Kopfprodukt 223 (entspricht Strom 9,153) der dritten Destillationskolonne 221 wird in die Kristallisationsvorrichtung 11 eingespeist. Dieses Kopfprodukt 223 enthält höhersiedende Komponente, welche durch Kristallisation eine gesättigte Carbonsäurekomponente, insbesondere in den FAME oder/und die gesättigte Fettsäure C18:0 und eine ungesättigte Carbonsäurekomponente, insbesondere in den FAME oder/und die ungesättigten Fettsäuren C18:1, C18:2 sowie C18:3 aufgetrennt wird.

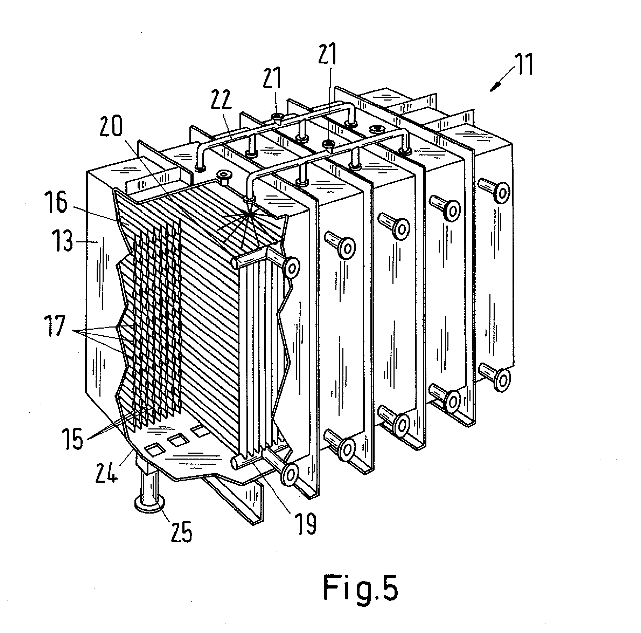

Fig. 5 zeigt eine Ansicht einer Kristallisationsvorrichtung zur Trennung von gesättigten und ungesättigten Carbonsäurekomponenten. Die Kristallisationsvorrichtung 11 weist einen Behälter 13 zur Aufnahme der Schmelze, welche die Carbonsäurekomponenten, das heisst das Produkt der Destillationsvorrichtung, nämlich die höhersiedende Komponente, insbesondere eine Fettsäure und/oder einen Fettsäuremethylester als gesättigt Carbonsäurekomponente, insbesondere als C18:0 oder als ungesättigte Carbonsäurekomponente, insbesondere als C18:1, C18:2, C18:3 enthält. In diesem Behälter 13 sind Wandelemente 15 angeordnet, die einen Abstand zueinander aufweisen. Die Wandelemente 15 enthalten geschlossene Kanäle 17 zur Zirkulation eines Temperiermittels. Durch Zirkulation des Temperiermittels im Inneren der geschlossenen Kanäle 17 ist jedes Wandelement 15 wahlweise heizbar oder kühlbar. Die geschlossenen Kanäle münden in einen Verteiler 19 und einen Sammler 20, welche zur Verteilung des Temperiermittels auf die einzelnen Kanäle 17 bzw. zur Aufnahme des Temperiermittels von den einzelnen Kanälen dienen. Die Zwischenräume 16 zwischen den Wandelementen 15 sind im Betrieb mit der Schmelze gefüllt, welche die zu trennenden Carbonsäurekomponenten enthält. Die Schmelze wird über Zuläufe 21, welche in Zulaufverteilelemente 22 münden, über die Gesamtheit der Wandelemente verteilt, sodass die Wandelemente 15 vollumfänglich von Schmelze umgeben sind. Nach dem Befüllen der Kristallisationsvorrichtung 11 mit Schmelze wird das Temperiermittel als Kühlmittel durch die Kanäle 17 geleitet, wodurch die Wandelemente 15 gekühlt werden. An den Wandelementen 15 kristallisiert die Schmelze zu einer Kristallisatschicht, die in ihrer Dicke kontinuierlich zunimmt. Aufgrund der unterschiedlichen Schmelzpunkte der einzelnen Carbonsäurekomponenten in der Schmelze enthält die Kristallisatschicht einen höheren Anteil an hochschmelzenden Carbonsäurekomponenten. Die Carbonsäurekomponenten mit den höchsten Schmelzpunkten lagern sich zu Beginn an den Kristallisationsflächen 39 der Wandelemente 15 ab, sind also in den wandnahen Schichten angereichert. Wird die Schmelze weiter abgekühlt, beginnen auch Carbonsäurekomponenten mit etwas niedrigeren Schmelzpunkten auszukristallisieren. Ein grosser Teil der niedrigstschmelzenden Carbonsäurekomponenten bleibt in der flüssigen Phase und wird über Abläufe abgelassen, die sich im Bodenbereich 24 der Kristallisationsvorrichtung 11 befinden. Die flüssige Phase wird auch als Mutterlauge bezeichnet. Sollen gesättigte und ungesättigte Carbonsäurekomponenten voneinander getrennt werden, werden die bei tieferer Temperatur schmelzenden, ungesättigten Carbonsäurekomponenten in der Mutterlauge angereichert. Die Mutterlauge enthält in diesem Fall das Wertprodukt. Bei Trennung der gesättigten Fettsäuren Stearinsäure von den ungesättigten Fettsäuren Ölsäure (C 18:1) Linolsäure (C 18:2) und Linolensäure (C 18:3) liegt der Schmelzpunkt von Stearinsäure bei ungefähr 70°C während der Schmelzpunkt von Ölsäure bei ca. 20°C und die Schmelzpunkte von Linolsäure und Linolensäure unter 0°C liegen. Sollten sich im Einsatzgemisch noch Reste von den gesättigten Fettsäuren C16, C14, C12 befinden, würden alle diese Fettsäuren gemeinsam mit der Stearinsäure als Kristallisat angelagert werden. Durch die starke Differenz der Schmelzpunkte zwischen gesättigten und ungesättigten Fettsäuren können die gesättigten und ungesättigten Fettsäuren mittels Kristallisation sehr effektiv getrennt werden.

In der zweiten Phase der Kristallisation werden die Wandelemente 15 wieder erwärmt. Während dieser zweiten Phase erfolgt ein teilweises Aufschmelzen der Kristallisatschicht, die sogenannte Schwitzphase. Während der Schwitzphase kann eine Fraktion der ungesättigten Carbonsäurekomponenten selektiv abgetrennt werden. Die Kristallisatschicht bleibt in der Schwitzphase mit den Wandelementen im wesentlichen verbunden, nur einzelne Tropfen werden abgezogen. In diesen ersten Tropfen ist die niedrigstschmelzende Carbonsäurekomponente, die gerade noch auskristallisiert ist, angereichert. In der Schwitzphase ist somit eine sehr selektive Trennung einer bestimmten Carbonsäurekomponente mit einem Schmelzpunkt, der zwischen den Schmelzpunkten der höchstschmelzenden Carbonsäurekomponente und der niedrigstschmelzenden Carbonsäurekomponente liegt, möglich. Während der Schwitzphase nimmt die Temperatur auf der Oberfläche der Wandelemente bevorzugt kontinuierlich zu. In diesem Fall können während der Schwitzphase auch mehrere Fraktionen abgezogen werden. In der dritten Phase erfolgt das Abschmelzen der Kristallisatschicht, also die Entfernung des Kristallisats von den Wandelementen 15. Hierzu werden die Kanäle 17 in den Wandelementen 15 von einem als Heizmittel wirkenden Temperiermittel durchströmt.

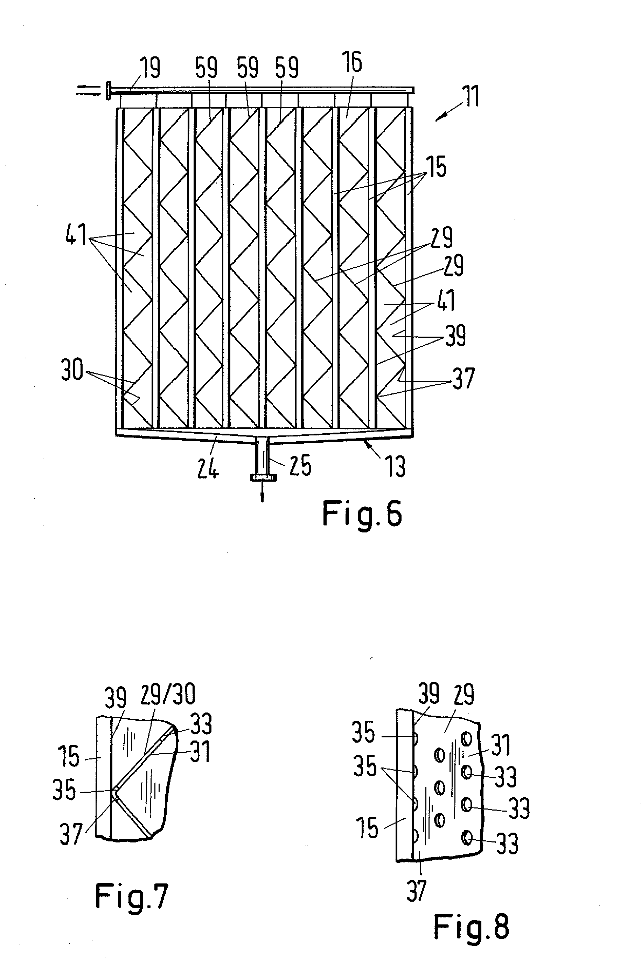

Fig. 6 zeigt einen Schnitt durch die Kristallisationsvorrichtung gemäss Fig. 5, in welcher zwischen den Wandelementen 15 siebartige Stützstrukturen 29 angeordnet sind. Diese Stützstrukturen 29 können aus Lochblechen gefertigt sein, wie in Fig. 7 oder Fig. 8 gezeigt ist. Die Lochung 33,35 der Lochbleche 31 dient der Durchlässigkeit der siebartigen Stützstrukturen 29 für die flüssige Phase. Die Stützstruktur 29 weist eine periodisch sich wiederholende Struktur auf. Diese periodisch sich wiederholende Struktur berührt abwechselnd das linksseitig und das rechtsseitig dargestellte Wandelement 15,welches an die Stützstruktur angrenzt. In der Fig. 6 oder Fig. 7 ist das Lochblech in einer Zickzackstruktur gefaltet, so dass benachbarte Kanten gegenüberliegende Wandelemente 15 längs der entsprechenden Kristallisationsfläche 39 berühren. Vorteilhafterweise ist eine Lochung an der die Kristallisationsfläche 39 berührenden Lochblechkante 37 angeordnet, damit auch in den am tiefsten liegenden Bereichen der in Fig. 6 dargestellten dreieckförmigen Zwischenräume 41 zwischen Stützstruktur und Kristallisationsfläche flüssige Phase ablaufen kann, was in Fig. 8 ersichtlich ist. Diese Lochung kann auch schlitzförmig ausgestaltet sein, wobei die Länge der Schlitze zumindest gleich gross wie die Kristallisatschicht ist, sodass die sich während der Schwitzphase bildenden Tropfen entlang der auf der Kristallisationsfläche 39 vorerst verbleibenden Kristallisatschicht entlang fliessen können, um im Bodenbereich gesammelt zu werden.

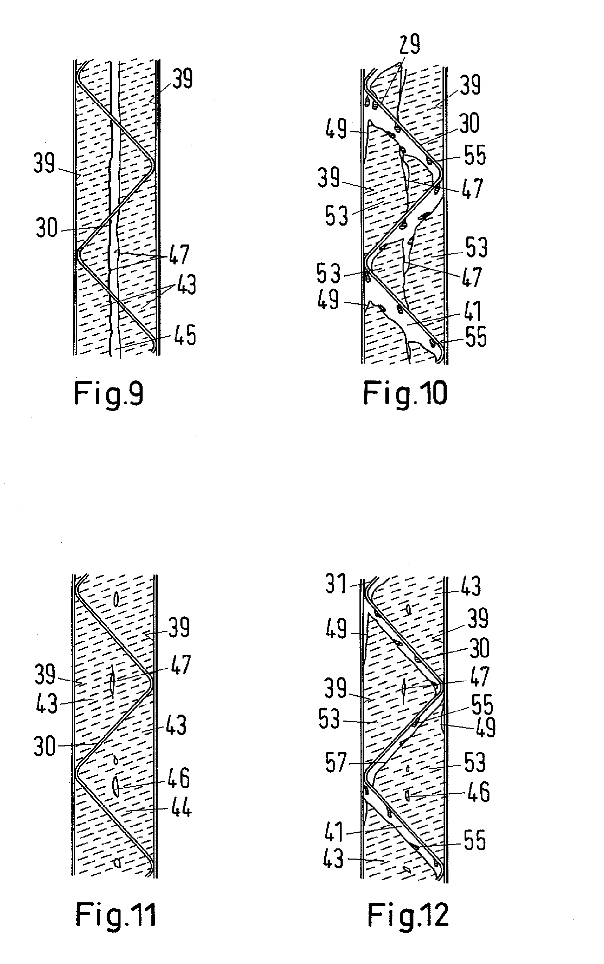

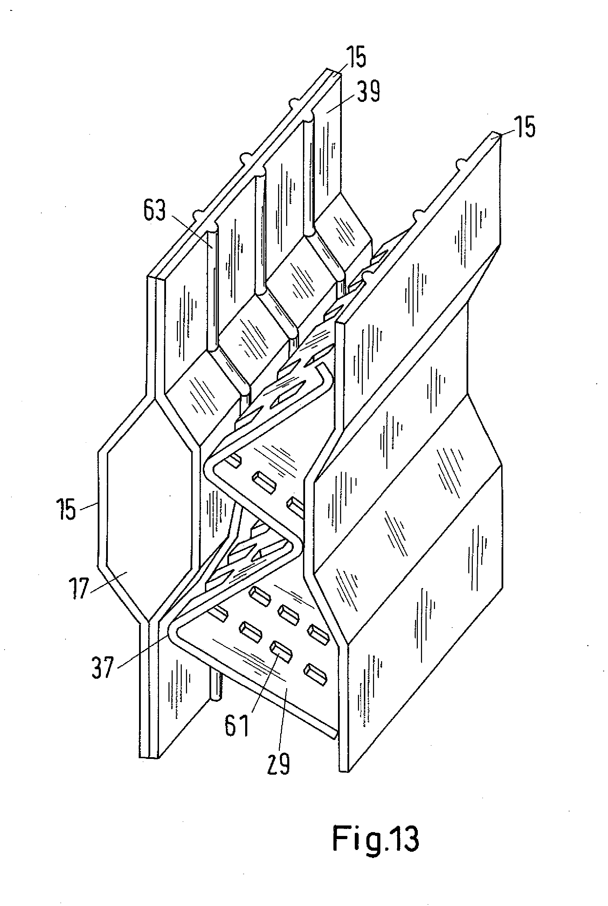

Fig. 9-12 zeigen die verschiedenen Stadien der Kristallisation in einer Kristallisationsvorrichtung gemäss einer der Fig. 5-8. Wird die Kristallisationsvorrichtung 11 zur Trennung von gesättigten und ungesättigten Carbonsäurekomponenten verwendet, so bildet sich während der ersten Phase des Kristallisationsverfahrens, der eigentlichen Kristallisation, auf den gekühlten Kristallisationsflächen 39 eine Kristallisatschicht 43. Zwischen zwei benachbarten Kristallisatschichten 43 kann ein Zwischenraum 45 verbleiben, in welchem die Mutterlauge als flüssige Phase in Richtung des Bodenbereichs 24 abströmen kann. Alternativ dazu können auch die beiden Kristallisatschichten 43 zu einer einzigen Schicht 44 zusammenwachsen, wobei die Schicht 44 Einschlüsse 46 enthalten kann, wie in Fig. 11 und Fig. 12 dargestellt ist. Während der zweiten Phase des Kristallisationsverfahrens, der Schwitzphase, wird zu Beginn die niedrigschmelzende Fraktion der Carbonsäurekomponenten ausgeschwitzt, das heisst in die flüssige Phase in die Form von Tropfen übergeführt. Diese Tropfen perlen von der Kristallisatschicht 43 ab und fliessen bevorzugt längs der Stützstruktur 29 ab, was in Fig. 10 gezeigt ist. Die Kristallisatschicht 43 wird durch die Erwärmung aufgeweicht, wodurch ihre Haftfähigkeit an der Kristallisationsfläche 39 reduziert ist. Spätestens zu dem Zeitpunkt, an dem die haftenden Oberfläche 49 der Kristallisatschicht 43 angeschmolzen ist, lösen sich Teile 53 der Kristallisatschicht 43 von der Kristallisationsfläche 39, die sich dann in der Stützstruktur verfangen. Diese Ablösung der Kristallisatschicht von der Kristallisationsfläche kann erwünscht sein, da sich einerseits der Wärmeübergang verbessert und andererseits die schwitzfähige Oberfläche der Kristallisatschicht 43 vergrössert werden kann. Die flüssige Phase kann gemäss Fig. 9 entlang der zentralen Oberfläche 47 sowie längs der Kristallisationsfläche 39 oder auch längs der Stützstruktur 29 in Richtung des Bodenbereichs 24 (siehe Fig. 6) strömen. Das Austreiben der flüssigen Phase wird durch den Druck des Eigengewichts des verbliebenen Kristallisatteils 53 begünstigt. Die flüssige Phase 55 tropft somit vom Kristallisatteil ab in die von der Stützstruktur 29 gebildeten kanalförmigen Zwischenräume sowie entlang der Kristallisationsfläche. Das Abströmen wird durch die Lochung 33, 35 oder die schlitzförmigen Öffnungen im Bereich der Kanten 37 des die Stützstruktur 29 bildenden Lochblechs 31 begünstigt. Die flüssige Phase 55 umströmt somit allseitig die Kristallisatteile 53, weil die flüssige Phase, die von oberhalb gelegenen Stützstrukturen auf das Kristallisatteil 53 fällt, über die geneigte Oberfläche 57 des Kristallisatteils 53 abläuft und durch die Lochungen 33, 35 der Stützstruktur 29 auf das unterhalb liegende Kristallisatteil tropft, wobei die Stützstruktur oder die Kristallisationsflächen Stege oder Rillungen aufweisen können, längs derer Tropfen koaleszieren können und Rinnsale ausbilden können, sodass abfliessende Flüssigkeit nicht von Kristallisatteilen behindert ist, was in Fig. 13 im Detail dargestellt ist.

In Fig. 13 ist ein Ausschnitt zu einem weiteren Ausführungsbeispiel der Kristallisationsvorrichtung gezeigt. Gemäss dieses Ausführungsbeispiels enthalten die Stützstrukturen 29, welche zwischen zwei benachbarten Wandelementen 15 angeordnet sind, anstatt einer Lochung schlitzförmige Ausnehmungen 61. Insbesondere an den Kanten 37 der Stützstruktur sind schlitzförmige Ausnehmungen angebracht, welche um die Kante herum verlaufen. Diese schlitzförmigen Ausnehmungen dienen dem Ablauf der flüssigen Phase.

Als weitere Modifikation weisen die Wandelemente Vorsprünge 63 auf, auf welchen die Stützstruktur 29 gelagert ist. Diese Variante gewährleistet, dass zwischen dem Wandelement 15 und der Stützstruktur 29 ein Abstand bestehen bleibt, da die Stützstruktur 29 nur an den Vorsprüngen 63 auf dem Wandelement aufliegt. Dieser Abstand hat mehrere Vorteile. Das Kristallwachstum auf der Kristallisationsfläche 39 kann während der Kristallisationsphase ungehindert erfolgen. Des weiteren kann während der Schwitzphase flüssige Phase 55 längs der Vorsprünge 63 sowie in dem Zwischenraum zwischen benachbarten Vorsprüngen 63, die Rillen ausbilden, und der Kante 37 der Stützstruktur in Richtung des Bodenbereichs 24 strömen. Somit kann die Strömung der flüssigen Phase längs der Kristallisationsfläche 39 ungehindert erfolgen, sodass die flüssige Phase schneller aus der Kristallisationsvorrichtung abziehbar ist. Somit kann die Dauer der Schwitzphase reduziert werden, was zur Folge hat, dass auch die gesamte Zykluszeit des diskontinuierlich ablaufenden Kristallisationsverfahrens reduziert werden kann. Zudem wird der Wärmeübergang zwischen dem im Kanal 17 strömenden Temperiermittel und dem Kristallisat verbessert, da sich zwischen Kristallisatteilen 53 und der Kristallisationsfläche 39 in weiten Bereichen ein Rinnsal oder sogar ein Flüssigkeitsfilm ausbildet der in der Schwitzphase durch die erhöhte Strömungsgeschwindigkeit zu einem verbesserten Wärmeübergang führt.

## Patentansprüche

1. Anlage zur Trennung von Carbonsäurekomponenten, umfassend eine Destillationsvorrichtung, in welcher die Carbonsäurekomponenten in eine leichtsiedende Komponente, eine höhersiedende Komponente und eine höchstsiedende Komponente zerlegbar sind, wobei die höhersiedende Komponente gesättigte oder ungesättigte Fettsäuren mit 18 Kohlenstoffatomen deren Siedepunkt bei 10 Torr zwischen 220 und 230°C liegt, und wobei die höchstsiedende Komponente mehr als 18 Kohlenstoffatome aufweist und gesättigte oder ungesättigte Fettsäuren mit mindestens 20 Kohlenstoffatomen umfasst, deren Siedepunkte bei 10 Torr über 230°C liegen, wobei der Destillationsvorrichtung eine Kristallisationsvorrichtung zur Trennung der höhersiedenden Komponente in gesättigte und ungesättigte Carbonsäurekomponenten nachgeschaltet ist, **dadurch gekennzeichnet, dass** die Kristallisationsvorrichtung als eine Schichtkristallisationsvorrichtung ausgebildet ist,
wobei die Schichtkristallisationsvorrichtung senkrechte oder geneigte Kristallisationsflächen zur Bildung eines Kristallisats an hochschmelzenden gesättigten Carbonsäurekomponenten enthält,
wobei die senkrechten oder geneigten Kristallisationsflächen zueinander in einem Abstand angeordnet sind, sodass eine Mutterlauge an niedrig schmelzenden ungesättigten Carbonsäurekomponenten abführbar ist, wobei eine für die flüssige Mutterlauge durchlässige siebartige Stützstruktur zwischen benachbarten Kristallisationsflächen vorgesehen ist, wobei stromaufwärts der Destillationsvorrichtung und der Kristallisationsvorrichtung eine Veresterungsvorrichtung angeordnet ist,
und wobei die Veresterungsvorrichtung einen Reaktor umfasst, der einen heterogenen Katalysator enthält.

2. Verfahren zur Trennung von Carbonsäurekomponenten, umfassend eine Destillationsvorrichtung in welcher in einem ersten Schritt die Carbonsäurekomponenten in eine leichtsiedende Komponente, eine höhersiedende Komponente und eine höchstsiedende Komponente, wobei die höhersiedende Komponente gesättigte oder ungesättigte Fettsäuren mit 18 Kohlenstoffatomen deren Siedepunkt bei 10 Torr zwischen 220 und 230°C liegt, und wobei die höchstsiedende Komponente mehr als 18 Kohlenstoffatome aufweist und gesättigte oder ungesättigte Fettsäuren mit mindestens 20 Kohlenstoffatomen umfasst, deren Siedepunkte bei 10 Torr über 230°C liegen, in einer Anlage nach Anspruch 1 zerlegt werden, in einem zweiten Schritt die höhersiedende Komponente einer Schichtkristallisationsvorrichtung zugeführt wird und in einem dritten Schritt die höhersiedende Komponente in der Schichtkristallisationsvorrichtung in ein Kristallisat, welches gesättigte Carbonsäurekomponenten enthält, und in eine Mutterlauge welche im wesentlichen ungesättigte Carbonsäurekomponenten enthält, getrennt wird.

3. Verfahren nach Anspruch 2, wobei die Carbonsäurekomponenten in einer Veresterungsvorrichtung zumindest teilweise in Carbonsäureester umgewandelt werden.

4. Verfahren nach Anspruch 3, wobei die Carbonsäureester bei Verwendung von Methanol im wesentlichen als Carbonsäuremethylester vorliegen, wobei bei Verwendung eines Einsatzgemisches aus Fettsäuren ein Fettsäureester als ein Fettsäuremethylester vorliegt.

5. Verfahren nach Anspruch 3, wobei die Veresterungsvorrichtung mittels heterogener Katalyse betrieben wird.

6. Verfahren nach einem der Ansprüche 2 bis 5 wobei die leichtsiedende Komponente ein Gemisch von mehreren Carbonsäurekomponenten mit unterschiedlicher Anzahl an Kohlenstoffatomen umfasst.

7. Verfahren nach Anspruch 6, wobei die leichtsiedende Komponente bis zu 16 Kohlenstoffatome enthält.

8. Verfahren nach Anspruch 7, wobei ein erster Teil des Gemischs der leichtsiedenden Komponenten in einer ersten Destillationskolonne (1,131) als Kopfprodukt abgezogen wird, ein zweiter Teil des Gemisches der leichtsiedenden Komponenten sowie die höhersiedende Komponente und die höchstsiedende Komponente als Sumpfprodukt der ersten Destillationskolonne (1,131) anfallen, wobei das Sumpfprodukt der ersten Destillationskolonne (1,131) einer zweiten Destillationskolonne (2, 141) zugeführt wird, wobei in der zweiten Destillationskolonne (2, 141) der zweite Teil des Gemisches der leichtsiedenden Komponenten als Kopfprodukt abgezogen wird, wobei die höhersiedende Komponente und die höchstsiedende Komponente als Sumpfprodukt der zweiten Destillationskolonne (2, 141) anfällt, wobei das Sumpfprodukt der zweiten Destillationskolonne (2, 141) einer dritten Destillationskolonne (3, 151) zugeführt wird, wobei die höhersiedende Komponente als Kopfprodukt der dritten Destillationskolonne (3, 151) der Kristallisationsvorrichtung (11) zugeführt wird, wobei als Sumpfprodukt der dritten Destillationskolonne (3, 151) die höchstsiedende Komponente abgezogen wird.

## Claims

1. A plant for the separation of carboxylic acid components comprising a distillation apparatus in which the carboxylic acid components can be broken down into a lower-boiling component, into a higher-boiling component and into an even higher-boiling component, wherein the higher-boiling component comprises saturated or unsaturated fatty acids having 18 carbon atoms whose boiling point at 10 Torr is between 220 and 230°C, and wherein the even higher-boiling component has more than 18 carbon atoms and comprises saturated or unsaturated fatty acids having at least 20 carbon atoms whose boiling points at 10 Torr are above 230°C, wherein a crystallization apparatus for the separation of the higher-boiling component into saturated and unsaturated carboxylic acid components is connected downstream of the distillation apparatus, **characterized in that** the crystallization apparatus is configured as a layer crystallization apparatus,
wherein the layer crystallization apparatus contains perpendicular or inclined crystallization surfaces for the formation of a crystallizate of high-melting saturated carboxylic acid components;
wherein the perpendicular or inclined crystallization surfaces are arranged at a spacing from one another so that a mother liquor of low-melting unsaturated carboxylic acid components can be led off, with a screen-like support structure permeable for the liquid mother liquor being provided between adjacent crystallization surfaces, with an esterification apparatus being arranged upstream of the distillation apparatus and the crystallization apparatus;
and wherein the esterification apparatus comprises a reactor comprising a heterogeneous catalyst.

2. A method for the separation of carboxylic acid components comprising a distillation apparatus in which, in a first step, the carboxylic acid components are broken down in a plant in accordance with claim 1 into a lower-boiling component, a higher-boiling component and an even higher-boiling component, wherein the higher-boiling component comprises saturated or unsaturated fatty acids having 18 carbon atoms whose boiling point at 10 Torr is between 220 and 230°C, and wherein the even higher-boiling component has more than 18 carbon atoms
and comprises saturated or unsaturated fatty acids having at least 20 carbon atoms whose boiling points at 10 Torr are above 230°C; in a second step, the higher-boiling component is supplied to a layer crystallization apparatus; and, in a third step, the higher-boiling component in the crystallization apparatus is separated into a crystallizate which contains saturated carboxylic acid components and into a mother liquor which contains essentially unsaturated carboxylic acid components.

3. A method in accordance with claim 2, wherein the carboxylic acid components are converted in an esterification apparatus at least partly into carboxylic acid esters.

4. A method in accordance with claim 3, wherein, on the use of methanol, the carboxylic acid esters are substantially present as carboxylic acid methyl esters, with a fatty acid ester being present as a fatty acid methyl ester on the use of a feed mixture of fatty acids.

5. A method in accordance with claim 3, wherein the esterification apparatus is operated by means of heterogeneous catalysis.

6. A method in accordance with any one of the claims 2 to 5, wherein the lower-boiling component comprises a mixture of a plurality of carboxylic acid components with a different number of carbon atoms.

7. A method in accordance with claim 6, wherein the lower-boiling component contains up to 16 carbon atoms.

8. A method in accordance with claim 7, wherein a first portion of the mixture of the lower-boiling component is drawn off as an overhead product in a first distillation column (1, 131), a second portion of the mixture of the lower-boiling components as well as the higher-boiling component and the even higher-boiling component accumulate as a sump product of the first distillation column (1, 131), with the sump product of the first distillation column (1, 131) being supplied to a second distillation column (2, 141), wherein, in the second distillation column (2, 141), the second portion of the mixture of the lower-boiling components is drawn off as an overhead product, with the higher-boiling component and the even higher-boiling component accumulating as a sump product of the second distillation column (2, 141), wherein the sump product of the second distillation column (2, 141) is supplied to a third distillation column (3, 151), with the higher-boiling component being supplied as an overhead product to the third distillation column (3, 151) of the crystallization apparatus (11), with the even higher-boiling component being drawn off from the third distillation column (3, 151) as a sump product.

## Revendications

1. Installation pour la séparation de composants d'acides carboxyliques, incluant un dispositif de distillation dans lequel les composants d'acides carboxyliques peuvent être décomposés en un premier composant à bas point d'ébullition, un composant à point d'ébullition plus élevé et un composant à point d'ébullition encore plus élevé, dans laquelle le composant à point d'ébullition plus élevé comprend des acides gras saturés ou insaturés avec 18 atomes de carbone dont les points d'ébullition sont entre 220 et 230° C à une pression de 10 Torr, et dans laquelle le composant à point d'ébullition encore plus élevé comprend plus de 18 atomes de carbone et inclut des acides gras saturés ou insaturés avec au moins 20 atomes de carbone, dont les points d'ébullition sont supérieurs à 230° C à une pression de 10 Torr, dans laquelle le dispositif de distillation est suivi d'un dispositif de cristallisation pour séparer le composant à point d'ébullition plus élevé en composants d'acides carboxyliques saturés et insaturés,
**caractérisée en ce que** le dispositif de cristallisation est réalisé comme un dispositif de cristallisation par couche,
dans laquelle le dispositif de cristallisation par couche contient des surfaces de cristallisation verticales ou inclinées pour former un dépôt cristallisé sur des composants d'acides carboxyliques saturés à haut point de fusion,
dans laquelle les surfaces de cristallisation verticales ou inclinées sont agencées les unes par rapport aux autres à une distance, de sorte qu'il est possible d'évacuer une saumure mère de composants d'acides carboxyliques insaturés à bas point de fusion,
dans laquelle il est prévu entre des surfaces de cristallisation voisines une structure de soutien analogue à un tamis qui laisse passer la saumure mère liquide, dans laquelle un dispositif destiné à l'estérification est agencé en amont du dispositif de distillation et du dispositif de cristallisation,
et dans laquelle le dispositif d'estérification inclut un réacteur qui contient un catalyseur hétérogène.

2. Procédé pour la séparation de composants d'acides carboxyliques, incluant un dispositif de distillation dans lequel, dans une première étape, les composants d'acides carboxyliques sont décomposés en un composant à bas point d'ébullition, un composant à point d'ébullition plus élevé et un composant à point d'ébullition encore plus élevé, dans lequel le composant à point d'ébullition plus élevé comprend des acides gras saturés ou insaturés avec 18 atomes de carbone, dont les points d'ébullition sont entre 220 et 230° C à une pression de 10 Torr, et dans lequel le composant à point d'ébullition encore plus élevé comprend plus de 18 atomes de carbone et inclut des acides gras saturés ou insaturés avec au moins 20 atomes de carbone, dont les points d'ébullition sont supérieurs à 230° C à une pression de 10 Torr, dans une installation selon la revendication 1,
dans une seconde étape, le composant à point d'ébullition plus élevé est amené à un dispositif de cristallisation par couche et,
dans une troisième étape le composant à point d'ébullition plus élevé est séparé en un dépôt cristallisé qui contient des composants acides carboxyliques saturés et en une saumure mère qui contient essentiellement des composants d'acides carboxyliques insaturés.

3. Procédé selon la revendication 2, dans lequel les composants d'acides carboxyliques sont convertis dans un dispositif d'estérification au moins partiellement en esters d'acides carboxyliques.

4. Procédé selon la revendication 3, dans lequel les esters d'acides carboxyliques se présentent essentiellement sous forme de méthyl-esters d'acides carboxyliques lors de l'utilisation de méthanol, et se présentent sous forme d'un méthyl-ester d'acide gras lors de l'utilisation d'un mélange d'apport composé d'acides gras.

5. Procédé selon la revendication 3, dans lequel le dispositif d'estérification est amené à fonctionner au moyen d'une catalyse hétérogène.

6. Procédé selon l'une des revendications 2 à 5, dans lequel le composant à bas point d'ébullition inclut un mélange de plusieurs composants d'acides carboxyliques avec un nombre différent d'atomes de carbone.

7. Procédé selon la revendication 6, dans lequel le composant à bas point d'ébullition contient jusqu'à 16 atomes de carbone.

8. Procédé selon la revendication 7, dans lequel une première partie du mélange des composants à bas point d'ébullition est soustraite à titre de produit de tête dans une première colonne de distillation (1, 131), une seconde partie du mélange des composants à bas point d'ébullition ainsi que le composant à point d'ébullition plus élevé et le composant à point d'ébullition encore plus élevé retombent sous forme de produit dans la bâche de la première colonne de distillation (1, 131), dans lequel le produit dans la bâche de la première colonne de distillation (1, 131) est admis à une seconde colonne de distillation (2, 141) et dans la seconde colonne de distillation (2, 141) la seconde partie du mélange des composants à bas point d'ébullition est soustraite à titre de produit de tête, dans lequel le composant à point d'ébullition plus élevé et le composant à point d'ébullition encore plus élevé retombent sous forme de produit dans la bâche de la seconde colonne de distillation (2, 141), dans lequel le produit dans la bâche de la seconde colonne de distillation (2, 141) est admis à une troisième colonne de distillation (3, 151), dans lequel le composant à point d'ébullition plus élevé est soustrait à titre de produit de tête de la troisième colonne de distillation (3, 151) et admis au dispositif de cristallisation (11), et dans lequel le composant à point d'ébullition encore plus élevé est soustrait à titre de produit de la bâche de la troisième colonne de distillation (3, 151).
